(19) Europäisches Patentamt
European Patent Office
Office européen des brevets

(11) EP 2 891 485 B1

(12) **EUROPEAN PATENT SPECIFICATION**

(45) Date of publication and mention
of the grant of the patent:
**26.12.2018 Bulletin 2018/52**

(51) Int Cl.:
*A61L 31/04* (2006.01)     *A61L 31/14* (2006.01)
*A61L 31/16* (2006.01)     *A61K 9/16* (2006.01)
*A61K 9/127* (2006.01)     *A61P 27/00* (2006.01)

(21) Application number: **13833842.1**

(22) Date of filing: **30.08.2013**

(86) International application number:
**PCT/KR2013/007867**

(87) International publication number:
**WO 2014/035206 (06.03.2014 Gazette 2014/10)**

(54) **METHOD FOR PREPARING MICROSPHERES FOR EMBOLI, AND METHOD FOR PREPARING MICROSPHERES TO WHICH DRUG-CONTAINING CARRIER IS BOUND**

VERFAHREN ZUR HERSTELLUNG VON MIKROKÜGELCHEN FÜR EMBOLI UND VERFAHREN ZUR HERSTELLUNG VON MIKROKÜGELCHEN ZUR BINDUNG VON WIRKSTOFFHALTIGEN TRÄGERN DARAN

PROCÉDÉ DE PRÉPARATION DE MICROSPHÈRES POUR EMBOLES, ET PROCÉDÉ DE PRÉPARATION DE MICROSPHÈRES AUXQUELLES EST LIÉ UN VECTEUR CONTENANT UN MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2012   KR 20120096589**
**31.08.2012   KR 20120096590**

(43) Date of publication of application:
**08.07.2015   Bulletin 2015/28**

(73) Proprietor: **Chung-Ang University Industry Academic Cooperation Foundation Seoul 156-756 (KR)**

(72) Inventors:
• **LEE, Jae Hwi**
**Seoul 137-811 (KR)**
• **KWAK, Byung Kook**
**Seoul 156-791 (KR)**
• **KIM, Hyeong Min**
**Seoul 120-816 (KR)**
• **LEE, Ga Hyeon**
**Seoul 156-830 (KR)**

(74) Representative: **Dehns**
**St. Brides House**
**10 Salisbury Square**
**London EC4Y 8JD (GB)**

(56) References cited:
JP-A- 2007 504 267     JP-A- 2011 507 871
KR-A- 20090 088 126     KR-B1- 100 496 802
US-A- 4 857 319     US-A1- 2009 274 762
US-A1- 2011 293 672

• CROWE L M ET AL: "IS TREHALOSE SPECIAL FOR PRESERVING DRY BIOMATERIALS?", BIOPHYSICAL JOURNAL, CELL PRESS, US, vol. 71, no. 4, 1 October 1996 (1996-10-01), pages 2087-2093, XP008033008, ISSN: 0006-3495

**Description**

[Technical Field]

The present disclosure relates to a method of preparing microspheres for emboli, and a method of preparing microspheres to which a drug-containing nano-carrier is bound.

[Background Art]

Embolization or embolotherapy is technology in which a certain substance is inserted into a blood vessel to control the stream of blood in which oxygen and nutrients are delivered to tumor tissues, thereby treating a tumor. Generally, inserts suitable for emboli should be biocompatible, hydrophilic, non-toxic, and biodegradable. In this case, microspheres such as chitosan, starch, gelatin, albumin, and sodium alginate have been widely researched as a material for emboli. Particularly, polyvinyl alcohol (PVA) particles such as Contour (PVA commercially available from Target Corp., USA), and Ivalon (commercially available from Laboratoire Ingenor, Paris) have been used for several years as main microspheres for emboli, which have occupied at least 80 % of the market. However, the PVA particles have a problem in that it is difficult to obtain a uniform particle size due to an irregular shape, resulting in degraded effectiveness of embolization and various side effects.

Microspheres for emboli have been used for embolization, and also researched as a carrier for a drug, a gene, etc., and multifunctional microspheres for emboli containing a drug have an excellent anticancer treatment efficacy. Such microspheres have been used in a liquid when inserted into the human body, but are preferably maintained in a solid state for stable storage. A lyophilization process is most preferred as a method for this purpose.

The term "lyophilization" for drugs refers to a method of obtaining a dried substance, which includes freezing a material containing an aqueous solution or moisture, and sublimating the frozen material under reduced pressure to remove the moisture. In this case, samples having unstable biological activities may be stored at room temperature for a long period of time.

For the microspheres for emboli, the particles which are easily deformed and exhibit elasticity are already known to have a superior embolic effect (Trisacryl gelatin microspheres for therapeutic embolization, II: Preliminary Clinical evaluation in tumors and arteriovenous malformations, Beaujeux et al, Am J Neuroradiol, 1996; 17:541-548).

However, conventional microspheres have problems in that, since the particles do not easily recover after lyophilization, an embolic effect may be reduced due to irregular size and shape, and the drug release may not maintained due to degraded biological activities.

Meanwhile, there is an increasing interest in microspheres for emboli enabling the drug release. However, conventional microspheres are dipped in a drug solution and subjected to an ion exchange process so as to contain a drug, and also have a mechanism in which the drug is delivered by an ion exchange reaction between ions in the blood and drug ions in the microspheres when the microspheres are inserted into the human body. Therefore, the conventional microspheres have a problem in that it is impossible to control the drug release without adjusting the density of ions in the human body.

Chitosan is a substance for emboli that is widely used to contain a drug. Although chitosan is non-toxic, biocompatible, and biodegradable, it has a problem in that it is easily broken during a procedure in which it swells in blood vessels during a surgical treatment with emboli. Korean Registered Patent No. 10-0478227 discloses a method of preparing chitosan microspheres, which includes adding ethanol to chitosan microspheres, substituting, under vacuum, the chitosan microspheres twice to include an anticancer drug, adding doxorubicin hydrochloride, and adsorbing the doxorubicin hydrochloride by means of an ion exchange reaction between molecular chains of chitosan to fix the chitosan microspheres. However, such a method has a problem in that a relatively large amount of doxorubicin hydrochloride is lost when the chitosan microspheres are washed with distilled water after the doxorubicin hydrochloride is adsorbed onto the chitosan microspheres, which makes it difficult to adjust the content of a drug.

Also, Korean Registered Patent No. 10-1157260 provides a water-insoluble and water-expandable synthetic anionic polymer using polyvinyl alcohol particles capable of containing a drug as a composition for chemoembolization. The patent document discloses that the synthetic anionic polymer is dipped in an aqueous doxorubicin solution for 24 hours so that doxorubicin is included in the microspheres using an ion exchange reaction, and then used for chemoembolization. However, the synthetic anionic polymer also has a problem in that the release of the drug may not be precisely controlled in the human body.

US2009/0274762 describes microspheres for use in embolic compositions.

US2011/293672 describes a composition for treating a pancreatic tumor or cyst comprising microspheres.

Crowe LM *et al*, 71, 4, 1996-10-01, describes the use of trehalose to stabilize liposomes during freeze-drying.

Therefore, there is a demand for a lyophilization method to allow the microspheres to maintain the physical stability and initial drug release after a lyophilization process. Further, there is also a demand for development of new microspheres capable of precisely controlling the drug release and having excellent physical properties.

[Disclosure]

[Technical Problem]

The present disclosure is designed to solve the problems of the prior art, and therefore the present inventors have tried to provide an optimized lyophilization method capable of maintaining physical properties and a drug release profile of particles intact even after microspheres for surgical treatment with emboli are lyophilized, and found that the microspheres recover to a level similar to that before lyophilization using trehalose as a lyophilization protectant and vortexing as a rehydrating method.

Also, described herein is a method of preparing an insert suitable for emboli, which is able to precisely control the drug release and simultaneously has physicochemically stable properties, and found that chitosan microspheres to which a drug-containing nano-carrier is bound are prepared by loading a drug in a nano-carrier and binding the nano-carrier to chitosan microspheres by means of an emulsification-crosslinking method, and the release of the drug from the prepared chitosan microspheres is precisely controlled according to a ratio of the drug and chitosan added. Therefore, the present disclosure has been completed, based on these facts.

That is, it is an object of the present disclosure to provide an optimized lyophilization method and a rehydrating method capable of allowing microspheres for emboli to exhibit recoverability after lyophilization. Described herein also are microspheres to which a drug-containing nano-carrier is bound, and which have a characteristic of precisely controlling the release of a drug in the human body by loading a drug for treating cancer in a nano-carrier to prepare a drug-containing nano-carrier and binding the nano-carrier to microspheres using an emulsification/crosslinking action.

However, a technical object to be achieved by the present disclosure is not limited to the above-described object, and other objects which are not mentioned above will be clearly understood by those skilled in the art.

[Technical Solution]

According to an aspect of the present disclosure, there is provided a method of preparing microspheres for emboli, which includes (a) adding trehalose to a microsphere suspension, (b) lyophilizing the suspension at a temperature in a range of -50 °C to -100 °C to prepare the lyophilized microspheres, and (c) adding the lyophilized microspheres to water or a buffer and vortexing the microspheres.

According to one exemplary embodiment of the present disclosure, the microspheres may be made of at least one material selected from the group consisting of chitosan, alginate, chitin, poly(N-isopropylacrylamide) (PNIPam), polyethylene glycol (PEG), poly(L-lactic acid) (PLLA), poly(D,L-lactic acid) (PDLLA), polyglycolic acid (PGA), polycaprolactone (PCL), polyhydroxyalkanoate, polydioxanone (PDS), polytrimethylene carbonate, poly(lactic acid-co-glycolic acid) (PLGA), poly(L-lactic acid-co-caprolactone) (PLCL), poly(glycolic acid-co-caprolactone) (PGCL), hyaluronic acid, chondroitin sulfate, dermatan sulfate, carboxymethyl cellulose, heparan sulfate, heparin, keratan sulfate, carboxymethyl hydroxyethyl cellulose, cellulose sulfate, cellulose phosphate, carboxymethyl guar, carboxymethyl hydroxypropyl guar, carboxymethyl hydroxyethyl guar, xanthan gum, Gellan gum, Welan gum, Rhamsan gum, agarose, furcellaran, pectin, gum arabic, gum tragacanth, carrageenan, starch phosphate, starch succinate, glycoaminoglycan, a polysaccharide, a polypeptide, an acrylamide, N-vinylpyrrolidone, dimethylacrylamide, acrylic acid, methacrylic acid, anhydric maleic acid, vinyl sulfonate, styrenecarboxylic acid, 2-acrylamido-2-methyl-propanesulfonic acid, vinylphosphonic acid, 2-methylacryloyloxyethyl sulfonic acid, gelatin, and collagen.

According to another exemplary embodiment of the present disclosure, the trehalose may be added to a content in a range of 3 % to 20 %, based on the volume of the microsphere suspension.

According to still another exemplary embodiment of the present disclosure, operation (c) may further include adding a contrast agent.

According to still another exemplary embodiment of the present disclosure, the contrast agent may be selected from the group consisting of a magnetic resonance imaging (MRI) contrast agent, a computed tomography (CT) contrast agent, a single photon emission computed tomography (SPECT) contrast agent, a positron emission tomography (PET) contrast agent, a bioluminescence (BL) contrast agent, an optical contrast agent, an X-ray contrast agent, and an ultrasonic contrast agent.

According to still another exemplary embodiment of the present disclosure, the vortexing may be performed for 5 minutes to 20 minutes.

According to still another exemplary embodiment of the present disclosure, a drug may be able to be released from the microspheres prepared by the method.

According to still another exemplary embodiment of the present disclosure, the drug may be a drug for treating cancer.

According to yet another exemplary embodiment of the present disclosure, the drug for treating cancer may be doxorubicin.

Described herein is a method of preparing chitosan microspheres, which includes (a) introducing a drug into a nano-carrier, (b) adding the nano-carrier having the drug introduced thereto to a chitosan solution to prepare a mixture solution and stirring the mixture solution, (c) adding the stirred mixture solution to a solution prepared by mixing an oil and an organic solvent, adding a surfactant, and stirring the mixture solution (an emulsification operation), and (d) adding glutaraldehyde-saturated toluene or genipin to the emulsified mixture solution dropwise and stirring the mixture solution (a crosslinking operation).

The nano-carrier may be selected from the group consisting of a liposome, a lipid nanoparticle, a nanocapsule, a nanoemulsion, and a nanostructure.

The nano-carrier may be a liposome.

The drug may be a drug for treating cancer.

The drug for treating cancer may be doxorubicin.

The oil may include at least one selected from the group consisting of paraffin oil, $\alpha$-bisabolol, stearyl glycyrrhetinate, salicylic acid, tocopheryl acetate, panthenol, glyceryl stearate, cetyl octanoate, isopropyl myristate, ethyl pelargonate, di-C12-13 alkyl malate, cetearyl octanoate, butylene glycol dicaptylate/dicaprate, isononyl isostearate, isostearyl isostearate, cetyl octanoate, octyldodecyl myristate, a cetyl esters, a C10-30 cholesterol/lanosterol ester, hydrogenated castor oil, a monoglyceride, a diglyceride, a triglyceride, beeswax, carnauba wax, sucrose distearate, PEG-8 beeswax, Candelilla (*Euphorbia cerifera*) wax, mineral oil, squalene, squalane, a monoglyceride, a diglyceride, a triglyceride, a medium-chain glyceride, miglyol, and cremophor.

The organic solvent may include at least one selected from the group consisting of petroleum ether, ethyl ether, isopropyl acetate, n-propyl acetate, isobutyl acetate, n-butyl acetate, isobutyl isobutyrate, 2-ethylhexyl acetate, ethylene glycol diacetate, C9 acetate, C10 acetate, methyl ethyl ketone, methyl isobutyl ketone, methyl isoamyl ketone, methyl n-amyl kiton, dibutyl ketone, cyclohexanone, isophorone, acetaldehyde, n-butyl aldehyde, croton aldehyde, 2-ethyl hexaaldehyde, isobutyl aldehyde, propion aldehyde, ethyl 3-ethoxypropionate, toluene, xylene, trichloroethane, propylene glycol mono methyl ethyl acetate, ethylene glycol monoethyl ether acetate, ethylene glycol monobutyl ether, diethylene glycol monobutyl ether acetate, dibutyl phthalate, diethyl phthalate, dimethyl phthalate, dioctyl phthalate, dioctyl terephthalate, butyl octyl phthalate, butyl benzene phthalate, dioctyl adipate, triethylene glycol di-2-ethylhexanoate, trioctyl trimethylate, glyceryl triacetate, glyceryl/tripropionin, and 2,2,4-trimethyl-1,3-pentanediol diisobutylate.

The surfactant may include at least one selected from the group consisting of sorbitan sesquioleate, glyceryl stearate, polysorbate 60, polysorbate 80, sorbitan triolein phosphate, sorbitan stearate, PEG-20 glyceryl isostearate, Ceteth-25, PEG-60 hydrogenated castor oil, Nonoxynol-15, PEG-6-decyltetradeceth-20, dimethicone copolyol, glyceryl diisostearate, Ceteth-24, cetearyl alcohol, polyoxyethylene nonylphenyl ether, PEG-40 hydrogenated castor oil, cetyl dimethicone copolyol, polyglyceryl-3 methylglucose distearate , PEG-100 stearate, sorbitan isostearate, sodium lauroyl glutamate, disodium cocoamphodiacetate, diethanolamine lauric acid, coconut fatty acid diethanolamide, N,N-bis-(2-hydroxyethyl)-cocamide, and cocamidopropyl betaine.

Described herein is a composition for surgical treatment with emboli, which includes chitosan microspheres, which are bound to a drug-containing nano-carrier using an emulsification-crosslinking method, as an active ingredient.

The drug may be a drug for treating cancer.

The drug for treating cancer may be doxorubicin.

[Advantageous Effects]

When the method of lyophilizing microspheres for emboli according to one exemplary embodiment of the present disclosure is used, the microspheres having stable physical properties can be prepared even after the lyophilization. The microspheres prepared by the method have advantages in that they can be efficiently used for embolization since they are in a uniform spherical form, have a diameter of 500 $\mu$m or less, and rapidly recover an original shape before the lyophilization when inserted into the human body.

Also, the microspheres for emboli treated by the method according to one exemplary embodiment of the present disclosure can be useful in being used as a delivery system from which an anticancer drug such as doxorubicin or cisplatin can be released.

In addition, unlike the products of the conventional art on which a process should be performed for an hour to 2 hours to load a drug onto the microspheres just before surgical treatment with emboli (for example, DC Beads commercially available from Biocompatibles Ltd., UK), the microspheres for emboli described herein have an advantage in that an additional time is not required to load a drug since the microspheres are prepared in a state in which the drug is loaded onto the microspheres from the beginning.

Ultimately, the microspheres for emboli according to one exemplary embodiment of the present disclosure have an advantage in that the microspheres are rapidly recover original physical properties even after lyophilization for stable storage, and have an anticancer effect by obstructing blood vessels in cancer cells with embolic particles, and an effective anticancer action due to the release of the anticancer drug.

Meanwhile, the conventional microspheres containing an anticancer drug are prepared using a method which includes coating the microspheres with an ion-exchange resin and chemically binding the ion-exchange resin to a drug so as to bind the anticancer drug to chitosan through ion exchange. The ion exchange method has problems in that a large amount of time is required, the amount of the introduced drug is not controlled, and a preparation process is very complicated.

Also, when the chitosan microspheres are prepared using the method disclosed in Korean Registered Patent No. 10-1058196 by the present inventors, the interaction between chitosan and the anticancer drug is relatively low. Therefore, the chitosan microspheres have a problem in that doxorubicin is easily lost while the chitosan microspheres are washed with distilled water.

However, the chitosan microspheres to which the drug-containing nano-carrier is bound as described herein have advantages in that, since the chitosan microspheres are bound to the drug-containing nano-carrier using an emulsification-crosslinking method, the amount of the drug lost during the washing process is remarkably reduced due to an increase in mutual viscosity, and the physical strength of the microspheres also increases.

Therefore, when the chitosan microspheres to which the drug-containing nano-carrier is bound as described herein, the chitosan microspheres have advantages in that the amount of the drug released from the chitosan microspheres can be more precisely controlled, and thus the drug release efficiency of the chitosan microspheres is also remarkably enhanced, and the chitosan microspheres have an excellent therapeutic effect in emboli due to an increase in physical strength of the chitosan microspheres.

Accordingly, since the chitosan microspheres to which the drug-containing nano-carrier is bound as described herein, exhibits the specificity of precisely controlling the drug release, when the chitosan microspheres are used to exhibit a superior effect, compared to the conventional drug release microspheres. Therefore, the chitosan microspheres can be useful in controlling the selective release of the drug against a target tumor, and can be used as a new insert suitable for emboli, which is used for anticancer treatment and have an excellent embolic effect.

[Description of Drawings]

FIG. 1 is a diagram showing the size distribution of particles to compare effects according to four types of lyoprotectants and effects according to a vortexing time;

FIG. 2 is a diagram showing the relationship between a mean diameter and sphericity of chitosan particles according to a vortexing time;

FIG. 3 is a diagram showing the results obtained by measuring weight fractions of particles whose particle size is recovered to an original size to compare effects according four types of lyoprotectants;

FIG. 4 is a diagram showing the results obtained by measuring weight fractions of particles whose particle size is recovered to an original size to compare effects according to a method of adding trehalose during a process, and a concentration of the trehalose;

FIG. 5 is a diagram showing the results obtained by an effect of loading of a drug on the size distribution of particles recovering after lyophilization;

FIG. 6 is a diagram showing the shapes of chitosan particles according to the loading of the drug before and after lyophilization (a magnification of 100 times);

FIG. 7 is a diagram showing the results obtained by performing a compression test using a texture analyzer to compare the elasticities of particles according to a vortexing process for recovering the particles;

FIG. 8 is a diagram showing the results obtained by performing a compression test using a texture analyzer to compare the elasticities of particles before and after lyophilization;

FIG. 9 is a diagram showing the results obtained by measuring a profile of doxorubicin released from chitosan particles before and after lyophilization;

FIG. 10 is a diagram showing the compositions and characteristics of a doxorubicin-containing liposome, doxorubicin-containing chitosan microspheres, and doxorubicin liposome-containing chitosan microspheres for emboli;

FIG. 11 is a diagram showing the optimum stirring rate (B) to obtain the doxorubicin-containing chitosan microspheres for emboli, and the size ratio (B) of the prepared chitosan microspheres; and

FIG. 12 is a diagram showing the amounts of doxorubicin released from the doxorubicin-containing chitosan microspheres and the doxorubicin liposome-containing chitosan microspheres according to the time elapsed.

[Best Mode]

Exemplary embodiments of the present disclosure will be described in detail below with reference to the accompanying drawings. While the present disclosure is shown and described in connection with exemplary embodiments thereof, it will be apparent to those skilled in the art that various modifications can be made without departing from the scope of the present disclosure.

Unless specifically stated otherwise, all the technical and scientific terms used in this specification have the same meanings as what are generally understood by a person skilled in the related art to which the present disclosure belongs. In general, the nomenclatures used in this specification and the experimental methods described below are widely known and generally used in the related art.

The present disclosure relates to an optimized lyophilization method and a rehydrating method capable of rapidly recovering microspheres for surgical treatment with emboli to an original shape before lyophilization and even after the lyophilization for stable storage of the microspheres.

The present inventors have conducted research on lyophilization protectants, lyophilization conditions, and a rehydrating method, which are optimal for chitosan microspheres, so as to establish the optimum lyophilization conditions which are not affected by the release of a drug and in which the chitosan microspheres may be easily used for embolization since the original shape of the chitosan microspheres is maintained intact even after a lyophilization process.

As a result, the present inventors have found that the chitosan microspheres are restored to a certain size and spherical shape similar to those of the chitosan microspheres before the lyophilization process when trehalose is added as a protectant and vortexed before the lyophilization process. Therefore, the present disclosure has been completed based on these facts.

Accordingly, the present disclosure is characterized in that it provides a method of preparing microspheres for emboli, which includes (a) adding trehalose to a microsphere suspension, (b) lyophilizing the suspension at a temperature in a range of -50 °C to -100 °C to prepare the lyophilized microspheres, and (c) adding the lyophilized microspheres to water or a buffer and vortexing the microspheres.

In the present disclosure, the term "vortex" or "vortexing" means that a fluid rotates about a certain center. According to one exemplary embodiment of the present disclosure, the vortexing is performed using a vortexer (VM-96B, Jeio Tech, Korea), but the present disclosure is not limited thereto.

In the present disclosure, the term "sphericity" refers to a degree of approximating a sphere by a particle, and is calculated as a ratio of the shortest length to the longest length of the particle. That is, the closer the sphericity is to 1, the more the particle approximates a sphere. Also, in the present disclosure, the term "substantially spherical" generally refers to a shape defined as a volume representing the minimum outer surface area, that is, refers to a shape approximating a perfect sphere. Specifically, in the present disclosure, the term "substantially spherical" means that a difference between a larger diameter and a smaller diameter is less than 20 %, less than 10 %, or less than 5 %, as viewed from any cross section of the microsphere.

To establish optimized lyophilization and recovery conditions for the microspheres for emboli, first, chitosan microspheres are prepared, and lyophilized according to one exemplary embodiment of the present disclosure. Here, the lyophilization is performed at a lyophilization temperature in the range of 50 °C to 100 °C below zero (-50 °C to -100 °C), preferably, a lyophilization temperature in a range of -60 °C to -80 °C, and most preferably, a lyophilization temperature of - 70 °C. In the present disclosure, the chitosan microspheres are lyophilized using a lyophilizer (FD 8508, Ilshin Biobase Co. Ltd, Dongduchun, Korea), but the present disclosure is not limited thereto.

To select a method of rehydrating the microspheres after lyophilization, first, physiological saline and a contrast agent were added, and a rehydrating method using a shaking constant-temperature water bath and a rehydrating method using vortexing were compared, and it was found that the rehydrating method using the vortexing is more preferred (see Example 1.3). The vortexing may be preferably performed for 5 minutes to 20 minutes, more preferably, for 8 minutes to 12 minutes, and most preferably, for 10 minutes.

Also, to select a lyoprotectant, glucose, lactose, sucrose, and trehalose were compared. As a result, it was found that a method, which includes preparing a microsphere suspension, adding trehalose to the microsphere suspension and lyophilizing the microsphere suspension, was most preferred (see Example 1.4). The trehalose may be preferably added at a content in a range of 3 % to 20 %, more preferably, a content of 10 %, based on the volume of the microsphere suspension.

The microspheres are made of a material that is biocompatible and having an embolic effect when inserted into the human body. Here, the material of the microspheres may be chitosan, alginate, chitin, poly(N-isopropylacrylamide) (PNIPam), polyethylene glycol (PEG), poly(L-lactic acid) (PLLA), poly(D,L-lactic acid) (PDLLA), polyglycolic acid (PGA), polycaprolactone (PCL), polyhydroxyalkanoate, polydioxanone (PDS), polytrimethylene carbonate, poly(lactic acid-co-glycolic acid) (PLGA), poly(L-lactic acid-co-caprolactone) (PLCL), poly(glycolic acid-co-caprolactone) (PGCL), hyaluronic acid, chondroitin sulfate, dermatan sulfate, carboxymethyl cellulose, heparan sulfate, heparin, keratan sulfate, carboxymethyl hydroxyethyl cellulose, cellulose sulfate, cellulose phosphate, carboxymethyl guar, carboxymethyl hydroxypropyl guar, carboxymethyl hydroxyethyl guar, xanthan gum, Gellan gum, Welan gum, Rhamsan gum, agarose, furcellaran, pectin, gum arabic, gum tragacanth, carrageenan, starch phosphate, starch succinate, glycoaminoglycan, a polysaccharide, a polypeptide, an acrylamide, N-vinylpyrrolidone, dimethylacrylamide, acrylic acid, methacrylic acid, anhydric maleic acid, vinyl sulfonate, styrenecarboxylic acid, 2-acrylamido-2-methylpropanesulfonic acid, vinylphosphonic acid, 2-methylacryloyloxyethyl sulfonic acid, gelatin, or collagen, preferably chitosan, alginate, or chitin, and more preferably, chitosan, but the present disclosure is not limited thereto.

The microspheres according to one exemplary embodiment of the present disclosure may further include a contrast agent to easily determine an embolic effect in blood vessels. In this case, a magnetic resonance imaging (MRI) contrast agent, a computed tomography (CT) contrast agent, a single photon emission computed tomography (SPECT) contrast agent, a positron emission tomography (PET) contrast agent, a bioluminescence (BL) contrast agent, an optical contrast agent, an X-ray contrast agent, or an ultrasonic contrast agent may be used as the contrast agent. Preferably, the contrast agent may be the X-ray contrast agent used in the present disclosure, but the present disclosure is not limited thereto.

According to one exemplary embodiment of the present disclosure, the microspheres for emboli having no drug loaded therein, and the microspheres for emboli having a drug loaded therein were also evaluated for recoverability so that the drug was able to be released from the microspheres prepared using the method according to one exemplary embodiment of the present disclosure (see Example 1.5).

Types of the "drug" used in the present disclosure are injected for anticancer embolization. Preferably, the drug may be a drug for treating cancer. The drug for treating cancer may include at least one selected from the group consisting of daunorubicin, adriamycin, doxorubicin, aclarubicin, epirubicin, valrubicin, idarubicin, mitoxantrone, mitomycin C, pirarubicin, rubidomycin, carcinomycin, N-acetyl adriamycin, rubidazone, daunoryline, bleomycin, cisplatin, dactinomycin, and paclitaxel. Most preferably, the drug for treating cancer may be the doxorubicin used in one exemplary embodiment of the present disclosure, but the present disclosure is not limited thereto.

From the above, the present disclosure may provide a lyophilization method capable of recovering the chitosan microspheres for emboli into an original shape before lyophilization in a state in which the chitosan microspheres are not affected by the physical properties and the drug release even after a lyophilization process.

In addition, the present inventors included a drug in a nano-carrier and bound the nano-carrier to the chitosan microspheres so as to conduct research on methods of preparing microspheres which may precisely control the drug release and are suitable for living bodies, as described above.

As a result, the present inventors have found that when the use of the chitosan microspheres having a drug-containing nano-carrier bound thereto makes it possible to more precisely control the content of the drug and significantly improve an effect of the chitosan microspheres on surgical treatment with emboli.

Therefore, described herein is a composition for surgical treatment with emboli including the drug-containing nano-carrier bound to the chitosan microspheres using an emulsification-crosslinking method as an active ingredient.

The term "emulsification-crosslinking method" used in the present disclosure refers to a method undergoing an emulsification process of stirring two or more solutions to form an emulsion and a crosslinking process of chemically forming covalent bonds in the formed emulsion.

It is confirmed that the composition for surgical treatment with emboli provided by the present disclosure may exhibit a swelling property in a pharmaceutically available solution, and may have a diameter in a range of 100 to 600 $\mu$m before swelling, which is suitable for the transfer of a drug.

Described herein is a liposome suspension is prepared using a liposome as the nano-carrier, and doxorubicin that is a drug for treating cancer is added thereto to prepare a doxorubicin liposome. Then, doxorubicin which is not loaded into the doxorubicin liposome is isolated, and measured for fluorescence. The amount of the unloaded doxorubicin may be determined from the measured value, and thus an exact amount of doxorubicin introduced to the liposome may be calculated (see Example 2.2).

Also, the doxorubicin liposome is bound to the microspheres using an emulsification-crosslinking method (see Example 2.3). In the above, since the exact amount of doxorubicin loaded into the liposome is determined, the amount of doxorubicin loaded onto the microspheres may also be determined.

The liposome and doxorubicin may be selected as the nano-carrier and the drug for treating cancer, respectively.

Therefore, described herein is a method of preparing chitosan microspheres, which includes:

> (a) introducing a drug into a nano-carrier;
> (b) adding the nano-carrier having the drug introduced thereto to a chitosan solution to prepare a mixture solution and stirring the mixture solution;
> (c) adding the stirred mixture solution to a solution prepared by mixing an oil and an organic solvent, adding a surfactant, and stirring the mixture solution (an emulsification operation); and
> (d) adding glutaraldehyde-saturated toluene or genipin to the emulsified mixture solution dropwise and stirring the mixture solution (a crosslinking operation).

The nano-carrier may be made of a lipid-based material or a polymeric material. In this case, the drug may be bound to a surface of the nano-carrier using a crosslinking agent. Therefore, the nano-carrier described herein is used as a drug delivery system. Here, the nano-carrier may be a liposome, a lipid nanoparticle, a nanocapsule, a nanoemulsion, or a nanostructure, preferably, a liposome or a lipid nanoparticle, and most preferably, a liposome.

The glutaraldehyde-saturated toluene or genipin used in the present disclosure may be used to crosslink the emulsion, any crosslinking agent may be used without limitation as long as it can crosslink the emulsion.

Types of the "drug" used in the present disclosure are introduced for anticancer embolization. Preferably, the drug may be a drug for treating cancer. Therefore, the drug for treating cancer may include at least one selected from the group consisting of daunorubicin, adriamycin, doxorubicin, aclarubicin, epirubicin, valrubicin, idarubicin, mitoxantrone, mitomycin C, pirarubicin, rubidomycin, carcinomycin, N-acetyl adriamycin, rubidazone, daunoryline, bleomycin, cisplatin, dactinomycin, and paclitaxel, and most preferably, doxorubicin.

The oil may be used to emulsify the nano-carrier and the chitosan microspheres, and preferably, includes at least one selected from the group consisting of paraffin oil, α-bisabolol, stearyl glycyrrhetinate, salicylic acid, tocopheryl acetate, panthenol, glyceryl stearate, cetyl octanoate, isopropyl myristate, ethyl pelargonate, di-C12-13 alkyl malate, cetearyl octanoate, butylene glycol dicaptylate/dicaprate, isononyl isostearate, isostearyl isostearate, cetyl octanoate, octyldodecyl myristate, cetyl esters, C10-30 cholesterol/lanosterol ester, hydrogenated castor oil, a monoglyceride, a diglyceride, a triglyceride, beeswax, carnauba wax, sucrose distearate, PEG-8 beeswax, Candelilla (*Euphorbia cerifera*) wax, mineral oil, squalene, squalane, monoglyceride, a diglyceride, a triglyceride, a medium-chain glyceride, miglyol, and cremophor, and most preferably, paraffin.

The organic solvent, may be a solvent used to mix the nano-carrier and the microspheres, may include at least one selected from the group consisting of petroleum ether, ethyl ether, isopropyl acetate, n-propyl acetate, isobutyl acetate, n-butyl acetate, isobutyl isobutyrate, 2-ethylhexyl acetate, ethylene glycol diacetate, C9 acetate, C10 acetate, methyl ethyl ketone, methyl isobutyl ketone, methyl isoamyl ketone, methyl n-amyl kiton, dibutyl ketone, cyclohexanone, isophorone, acetaldehyde, n-butyl aldehyde, croton aldehyde, 2-ethyl hexaaldehyde, isobutyl aldehyde, propion aldehyde, ethyl 3-ethoxypropionate, toluene, xylene, trichloroethane, propylene glycol mono methyl ethyl acetate, ethylene glycol monoethyl ether acetate, ethylene glycol monobutyl ether, diethylene glycol monobutyl ether acetate, dibutyl phthalate, diethyl phthalate, dimethyl phthalate, dioctyl phthalate, dioctyl terephthalate, butyl octyl phthalate, butyl benzene phthalate, dioctyl adipate, triethylene glycol di-2-ethylhexanoate, trioctyl trimethylate, glyceryl triacetate, glyceryl/tripropionin, and 2,2,4-trimethyl-1,3-pentanediol diisobutylate, and a mixture thereof, preferably, petroleum ether, ethyl ether, and isopropyl acetate, and most preferably, petroleum ether .

The surfactant may be added so that the chitosan microspheres, the nano-carrier, the oil and the organic solvent are readily mixed during an emulsification process, and may include at least one selected from the group consisting of sorbitan sesquioleate, glyceryl stearate, polysorbate 60, polysorbate 80, sorbitan triolein phosphate, sorbitan stearate, PEG-20 glyceryl isostearate, Ceteth-25, PEG-60 hydrogenated castor oil, Nonoxynol-15, PEG-6-decyltetradeceth-20, dimethicone copolyol, glyceryl diisostearate, Ceteth-24, cetearyl alcohol, polyoxyethylene nonylphenyl ether, PEG-40 hydrogenated castor oil, cetyl dimethicone copolyol, polyglyceryl-3 methylglucose distearate , PEG-100 stearate, sorbitan isostearate, sodium lauroyl glutamate, disodium cocoamphodiacetate, diethanolamine lauric acid, coconut fatty acid diethanolamide, N,N-bis-(2-hydroxyethyl)-cocamide, and cocamidopropyl betaine, preferably, sorbitan sesquioleate, glyceryl stearate, and sorbitan stearate, and most preferably, sorbitan sesquioleate.

Further, described herein is an experiment to compare the drug releases of the doxorubicin-containing chitosan microspheres and the doxorubicin liposome-containing chitosan microspheres. As a result, it is confirmed that the use of the chitosan microspheres containing a doxorubicin liposome makes it possible to precisely control the drug release (see Example 2.5).

From the above, described herein is a composition for surgical treatment with emboli including the chitosan microspheres, which are bound to the drug-containing nano-carrier using an emulsification-crosslinking method, as an active ingredient.

Hereinafter, preferred embodiments are provided to aid in understanding the present disclosure. However, it should be understood that detailed description provided herein is merely intended to provide a better understanding of the present disclosure, and is not intended to limit the scope of the present disclosure.

**Example 1: Preparation of microspheres for emboli**

**1.1. Preparation and lyophilization of chitosan microspheres**

According to one exemplary embodiment of the present disclosure, chitosan was used as a material of the microspheres for surgical treatment with emboli, and the chitosan microspheres was prepared using a method previously developed by the present inventors (Korean Registered Patent No. 10-1058196). First of all, chitosan (approximately 85 % of which was diacetylated) was dissolved in a 5 % (v/v) aqueous acetic acid solution to prepare a 4 % (w/v) chitosan solution. Thereafter, polyethylene glycol (PEG having a molecular weight of 20,000 g/mol) was added to the viscous chitosan solution so that PEG and the chitosan was mixed at a weight ratio (w:w) of 3:7, and mixed for 60 minutes while stirring until PEG was completely dissolved at room temperature. Doxorubicin was added to the solution, tripolyphosphate was added, and gelled for 60 minutes. A solution prepared by adding 6 ml of sorbitan sesquioleate as an emulsifying agent, and the chitosan/polyethylene glycol solution undergoing the gelling process were added to 60 ml of a solution prepared by mixing liquid paraffin and petroleum ether at a volume ratio of 7:5, and emulsified for 10 minutes while stirring at 1000 rpm. Then, 8 ml of glutaraldehyde-saturated toluene was added dropwise for crosslinking, and then stirred at 1000 rpm for 60 minutes. The crosslinked chitosan microspheres were washed three times with petroleum ether, once with acetone, and three times with water to remove the remaining organic solvents. The washed microspheres were maintained for 24 hours in a shaking constant-temperature water bath which was maintained at constant room temperature to form a porous structure, and hydrophilic polyethylene glycol was extracted. As a result, the spherical chitosan microspheres having a diameter of approximately 400 $\mu$m were prepared.

For the lyophilization according to one exemplary embodiment of the present disclosure, 3 ml of a chitosan microsphere suspension prepared by the above-described process was put into a 20 ml glass vial, frozen at -70 °C, and then dried in a lyophilizer(FD 8508, Ilshin Biobase Co. Ltd, Korea), and a lyoprotectant was added to the prepared microsphere suspension before lyophilization.

### 1.2. Evaluation of method of rehydrating lyophilized microspheres

To select the conditions which were most suitable for rehydrating the lyophilized chitosan microspheres, methods using a shaking constant-temperature water bath (BS-21, Jeio Tech, Korea) and vortexing (VM-96B, Jeio Tech, Korea) were evaluated according to the time elapsed. As a solution most frequently used to inject particles for emboli, a solution prepared by mixing physiological saline and an X-ray contrast agent (Iobrix injection, Taejoon Pharm Co. Ltd., Korea) at a volume ratio of 1:1 was added at the same volume of 3 ml as that before lyophilization, and the rehydration was then performed.

### 1.2.1. Results of method using shaking constant-temperature water bath

To evaluate the recoverability of the chitosan microspheres after the chitosan microspheres were rehydrated for 12 hours and 24 hours in a shaking constant-temperature water bath, the particle size distribution of the chitosan microspheres was evaluated using a sieve, and the shape of the particles was observed under a microscope, and the mean particle size and sphericity were determined. In this case, 50 chitosan microspheres were randomly selected, and measured for size and sphericity. The measurement was performed using image analysis software (Scopephoto, Hangzhou, China). The sphericity was calculated as a ratio of the longest length and the shortest length of the particles, and the closer the sphericity is to 1, the more the particle approximates a sphere. The mean size of the particles which were rehydrated at 50 rpm for 12 hours or 24 hours in a shaking constant-temperature water bath after lyophilization was smaller than that of the microspheres before the lyophilization, and the shape of the chitosan microspheres was highly irregular, indicating that the particles were slow to recover due to a slow water penetration rate.

### 1.2.2. Evaluation results of method using vortexing

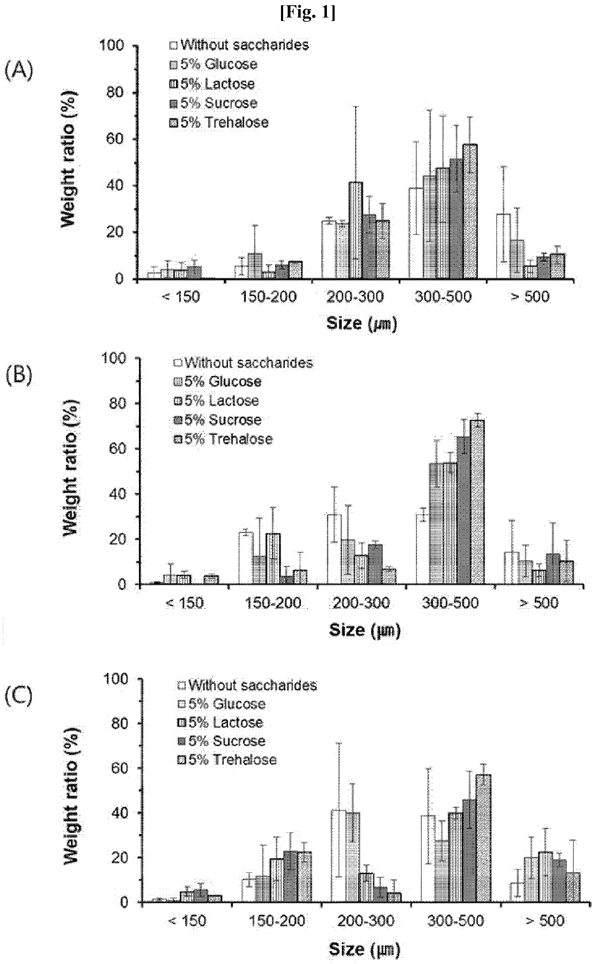

Effects according to a vortexing time, and effects according to the type of a lyoprotectant were compared, and are shown in FIG. 1. The size distribution of the chitosan microspheres vortexed for 5 minutes is shown in FIG. 1(A), and was uniformed between 200 to 500 $\mu$m, the size of which significantly differed from 400.5$\pm$42.0 $\mu$m of the chitosan microspheres before the lyophilization. FIG. 1B shows the results between 300 to 500 $\mu$m obtained by vortexing for 10 minutes. As a result, it could be seen that the chitosan microspheres after vortexing had a size similar to the original particle size of 400.5$\pm$42.0 $\mu$m before the lyophilization. FIG. 1C shows the results obtained by vortexing for 15 minutes. As a result, it was revealed that the particles sufficiently recover while being vortexed for 15 minutes excessively collided with each other, resulting in uneven sizes of the particles as a whole.

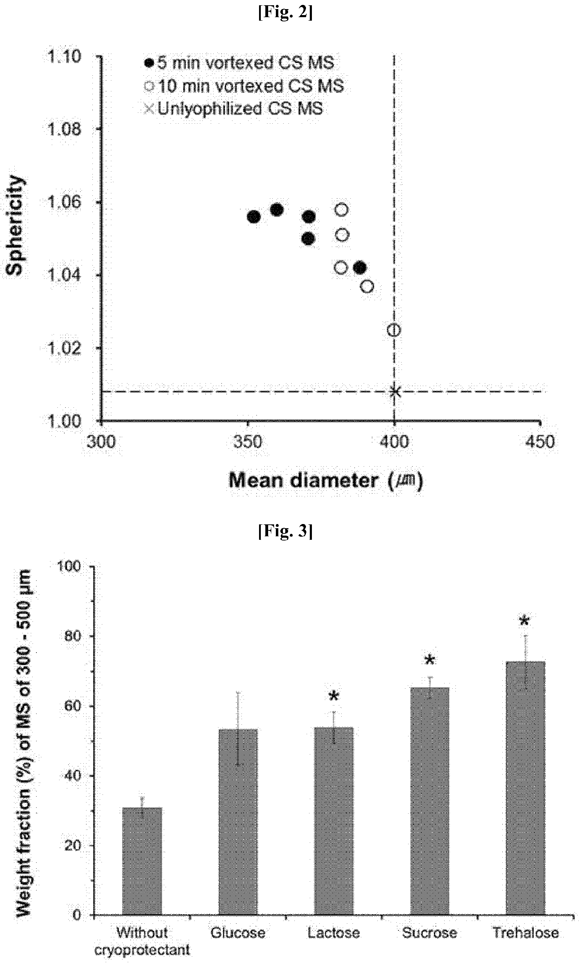

The sphericity and mean diameters of the chitosan microspheres according to the vortexing time were compared, and are shown in FIG. 2. The sphericity of the microspheres before the lyophilization was 1.008$\pm$0.015, to which the chitosan microspheres vortexed for 10 minutes had the closest value.

From the results, it was confirmed that it was most desirable to vortex the chitosan microspheres for 10 minutes as the method of recovering the chitosan microspheres.

### 1.3. Evaluation for selection of optimum lyoprotectant and concentration thereof, and preparation method

The most effective lyoprotectant was selected, and evaluations for selecting a method of adding the selected lyoprotectant, and the optimum concentration of the lyoprotectant were performed.

### 1.3.1. Evaluation results of recoverability according to type of lyoprotectant

To select the most effective lyoprotectant, four types of saccharides such as glucose, lactose, sucrose, and trehalose were added to a chitosan microsphere suspension at a final concentration of 5 % (w/v), and then lyophilized to be rehydrated. Thereafter, to evaluate the recoverability, the weight fractions of the particles which recovered a size of 300 to 500 $\mu$m, which fell within an original particle size of 400.5$\pm$42.0 $\mu$m, were measured using a sieve. The measurement results are shown in FIG. 3. Also, the shape of the microspheres was observed under a microscope, and the mean size and sphericity of the microspheres were measured. As shown in FIG. 3, it was revealed that, when the glucose was present at a content of 300 to 500 $\mu$m, the chitosan microspheres had a significant size recoverability of approximately 5 0%, which was not statistically significantly different from that of the chitosan microspheres to which the lyoprotectant was not added, and also that the chitosan microspheres had a significant size recoverability of 53 % and 65 %, respectively, when lactose and sucrose were added. Further, it was revealed that the particles to which trehalose was added had a very excellent recoverability of 73 %, compared to when the protectants were added. As listed in Table 1, it was confirmed that, when trehalose was used as the lyophilization protectant, the chitosan microspheres had the most excellent recoverability. Therefore, trehalose was used for the following evaluations.

Table 1

| | Vortexing for 5 minutes | | Vortexing for 10 minutes | | Vortexing for 15 minutes | |
|---|---|---|---|---|---|---|
| | Mean diameter ($\mu$m) | Sphericity | Mean diameter ($\mu$m) | Sphericity | Mean diameter ($\mu$m) | Sphericity |
| Before lyophilization | 400.5$\pm$42.0 | 1.008$\pm$0.015 | 400.5$\pm$42.0 | 1.008$\pm$0.015 | 400.5$\pm$42.0 | 1.008$\pm$0.015 |
| No cyoprotectant | 352.0$\pm$40.3 | 1.056$\pm$0.033 | 381.9$\pm$23.4 | 1.058$\pm$0.047 | 362.6$\pm$27.3 | 1.061$\pm$0.041 |
| 5 % Glucose | 359.8$\pm$44.0 | 1.058$\pm$0.04 | 382.2$\pm$27.3 | 1.051$\pm$0.039 | 362.4$\pm$28.1 | 1.058$\pm$0.044 |
| 5 % Lactose | 388.2$\pm$44.0 | 1.042$\pm$0.031 | 390.7$\pm$28.4 | 1.037$\pm$0.032 | 384.4$\pm$29.4 | 1.084$\pm$0.035 |
| 5 % Sucrose | 370.8$\pm$34.9 | 1.056$\pm$0.055 | 381.8$\pm$27.8 | 1.042$\pm$0.044 | 382.8$\pm$35.6 | 1.043$\pm$0.025 |
| 5 % Trehalose | 370.5$\pm$46.0 | 1.050$\pm$0.041 | 399.8$\pm$25.6 | 1.025$\pm$0.026 | 382.0$\pm$27.5 | 1.029$\pm$0.023 |

### 1.3.2. Evaluation results of recoverability according to method of adding trehalose

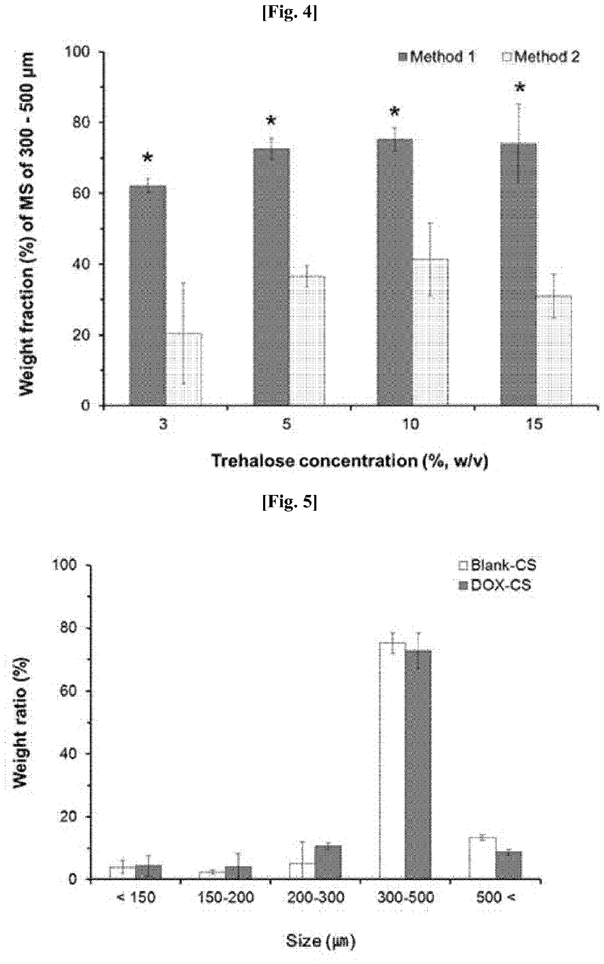

To compare a method of adding trehalose selected as the optimum lyoprotectant to the prepared chitosan microsphere suspension (method 1) and a method of adding trehalose during preparation of the chitosan microspheres (method 2), the weight fractions of the particles which recovered an original particle size were measured using a sieve, and are shown in FIG. 4. The shape of the particles was observed under a microscope, and the mean particle size and sphericity of the particles were measured. As a result, it was revealed that the chitosan microspheres having a diameter of 300 to 500 $\mu$m had a weight fraction in a range of 60 % to 75 % when the method of adding trehalose to the prepared chitosan particles suspension (method 1) was used, indicating that the chitosan microspheres exhibited significantly excellent recoverability. Also, it was revealed that the recoverability of the particles rather tended to decrease when the method of adding trehalose during a preparation process was used. Therefore, the following evaluations were performed using the method of adding trehalose to the suspension after preparation of the particles.

### 1.3.3. Evaluation results of recoverability according to concentration of trehalose

To evaluate the recoverability according to the concentration of trehalose, trehalose was added to the chitosan microsphere suspension at a concentration of 3, 5, 10, and 15 % (w/v), and then lyophilized to be rehydrated. Thereafter, the weight fractions of the particles recovering an original particle size were measured using a sieve. The shape of the particles was observed under a microscope, and the mean particle size and sphericity of the microspheres were measured. As shown in FIG. 4, it was revealed that the chitosan microspheres had similar fractions in a range of 72 % to 75 % at a trehalose concentration of 5 % or more when the fractions of the chitosan microspheres recovering an original particle size were measured. However, it was observed that the surface of the particles was much smoother and highly spherical at a trehalose concentration of 10 %, compared to that at a trehalose concentration of 5 %, when the shape of the particles was observed under a microscope. Since the recoverability were measured to be similar at the trehalose concentration of 10 % and 15 %, trehalose was used at a concentration of 10 % in the following evaluations.

### 1.3.4. Evaluation of recoverability according to loading of drug

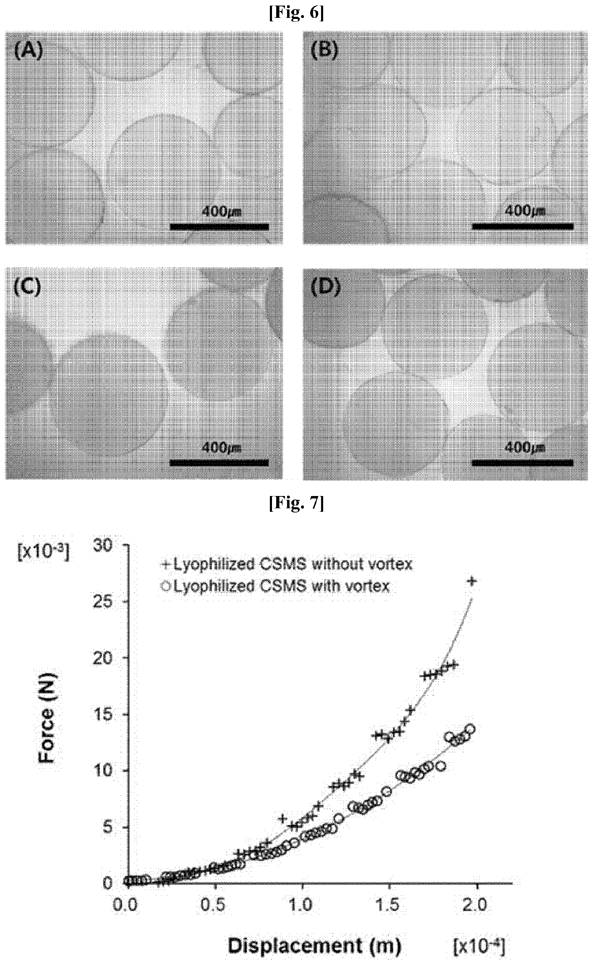

The particles for emboli having no drug loaded thereto and the particles having an anticancer drug doxorubicin loaded thereto were equally applied to the optimum process selected through the above-described evaluations to evaluate whether the chitosan microspheres had the same recoverability. First of all, the chitosan microspheres were observed under a microscope to evaluate the physical properties of the particles according to the loading of the drug before lyophilization. The results are shown in FIG. 6. As a result, it was revealed that the chitosan microspheres had all the same other physical properties, except that the particles having no drug loaded thereto were tinged with yellow, and the particles having the drug loaded thereto were tinged with red which is the innate color of doxorubicin. To evaluate the recoverability after lyophilization, the size distribution of the particles was evaluated using a sieve. The results are shown in FIG. 5. The shape of the particles was observed under a microscope, and the mean particle size and sphericity of the chitosan microspheres were measured. The results are shown in Table 2. As a result, it could be seen that the chitosan microspheres had a diameter of 300 to 500 $\mu$m even when the drug was loaded onto the chitosan microspheres, indicating that the loading of the drug had no influence on the lyophilization process and the recoverability, and the same results were obtained.

Table 2

|  | Chitosan microspheres | | Doxorubicin-containing chitosan microsopheres | |
| --- | --- | --- | --- | --- |
|  | Mean diameter ($\mu$m) | Sphericity | Mean diameter ($\mu$m) | Sphericity |
| Before lyophilization | 400.5$\pm$42.0 | 1.008$\pm$0.015 | 400.8$\pm$46.6 | 1.012$\pm$0.010 |
| After lyophilization | 401.5$\pm$35.1 | 1.028$\pm$0.019 | 391.7$\pm$16.9 | 1.037$\pm$0.022 |

### 1.4. Evaluation of elasticity of rehydrated chitosan microspheres

Based on the previous research showing that the particles which were easily deformed and exhibit elasticity had an excellent embolic effect, the elasticity of the particles before/after lyophilization was evaluated. To compare the elasticity of the chitosan microspheres, a compression test was performed using a texture analyzer (TA plus, Lloyd Instrument Ltd., UK). A texture analyzer probe having a flat cross section, a diameter of 5 mm and a length of 50 mm, and a 100 N load cell was used. Since the particles had a micro-scaled size, approximately 150 particles were evaluated in a state in which the particles were placed in physiological saline so as to prevent the particles from being dried due to exposure to the air when the particles were spread in a single layer. The evaluation started immediately before the texture analyzer probe came in contact with the particles, and the microspheres were compressed at a rate of 1 $\mu$m/s. Then, this experiment was performed until the diameter of the microspheres was deformed by 30 to 50 %. The total compressive force measured based on the previous research was divided by a total number of the particles to measure a compressive force applied to the respective particles.

### 1.4.1. Evaluation of elasticity of chitosan microspheres according to rehydrating method

To evaluate the elasticity of the particles according to a rehydrating method, trehalose was added to a particle suspension at a concentration of 10 %, and lyophilized. Thereafter, the compressive forces of the particles undergoing a vortexing process for 10 minutes as selected as the optimum rehydrating method, and the particles maintained in physiological saline for 10 minutes without any vortexing process were measured, and compared to evaluate the elasticity. The results are shown in FIG. 7. The compressive forces of the microspheres when the diameter of the microspheres was deformed by 20 % were measured. As a result, it was revealed that the compressive force of the particles undergoing the vortexing process for 10 minutes was measured to be 2.96$\pm$0.40 ($\times 10^{-3}$ N), and the compressive force of the particles maintained for 10 minutes was measured to be remarkably high at 6.17$\pm$1.04 ($\times 10^{-3}$ N). The higher compressive force means harder particles, which was in inverse proportion to the elasticity of the particles. As a result, it was contemplated that the particles which did not undergo a rehydration process had poor deformability which was a condition required upon surgical treatment with emboli. The results showed that the particles undergoing the vortexing process were much smoother and the elasticity of the particles was recovered. In a profile of a change in compressive force according to the displacement, the physical properties of the particles remarkably differed according to the presence of the rehydration process. Therefore, it could be seen, once again, that the recovery of the particles was promoted when the vortexing process was performed for 10 minutes.

**1.4.2. Comparison and evaluation results of elasticity of chitosan microspheres before and after lyophilization**

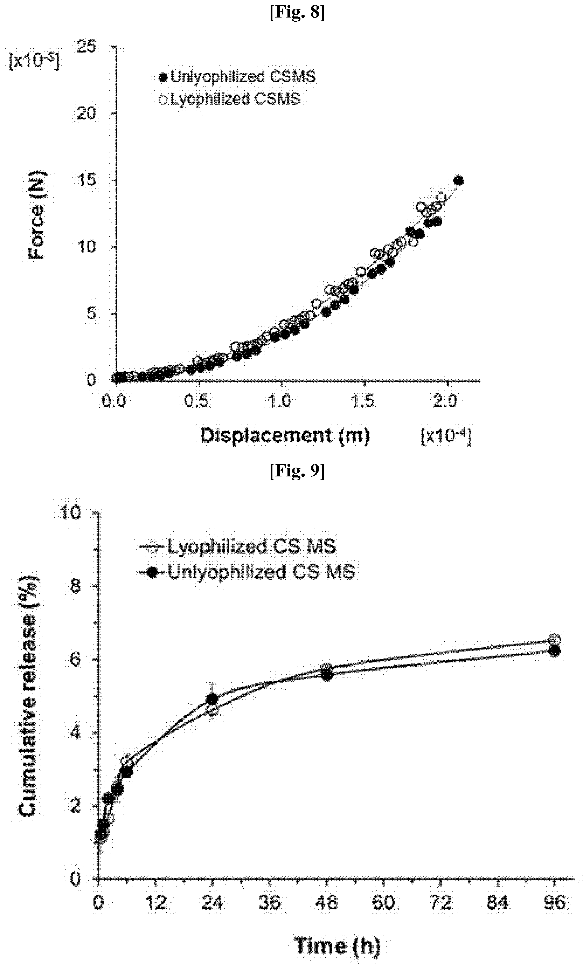

This object of the evaluation was to determine whether the physical properties of the particles undergoing the inventive process of optimizing the recoverability recovered to a level similar to those of the particles before the lyophilization. The results are shown in FIG. 8. The compressive force of the microspheres when the diameter of the microspheres was deformed by 20 % was measured. As a result, it was revealed that the compressive force of the particles before the lyophilization was measured to be $2.56 \pm 0.30$ ($\times 10^{-3}$ N), and the compressive force of the particles recovering after the lyophilization was measured to be slightly high at $2.96 \pm 0.40$ ($\times 10^{-3}$ N), but had no statistically significant difference. That is, the particles were able to be proven to recover into an original state without any change in elasticity. Also, changes in sphericity before and after the compression test were measured. As a result, it was revealed that there was no statistically significant change in the sphericity, indicating that the particles recovered an original shape due to the elasticity of the particles after the compression test.

**1.5. Comparison evaluation of drug release profiles of chitosan microspheres before and after lyophilization**

Phosphate buffered saline (pH 6.0) including 140,000 units of a lysozyme was used as a medium for evaluating a drug release profile, and the drug release profile were observed for 4 days. 150 mg of the particles was added into a dialysis bag (MW cut-off = 12,000 to 14,000), and the dialysis bag was put into a conical tube containing 30 ml of a medium, maintained at a temperature of $37 \pm 0.5$°C in a shaking constant-temperature water bath, and shaken at 80 rpm. 0.2 ml of a release medium was taken at intervals of 0.5, 1, 2, 4, and 6 hours, and 1, 2, and 4 days, and analyzed. In this case, 0.2 ml of a medium having the same compositions was added right after the release medium was taken. The analysis of released doxorubicin was performed using a microplate reader (Synergy HI Hybrid Reader, Bio Tek, Korea), and the measurement was performed at an excitation wavelength of 480 nm and an emission wavelength of 550nm. The results are shown in FIG. 9. As a result, it was revealed that the drug release profiles of the particles before the lyophilization, which were taken right after the preparation of the particles, and the particles recovering after the lyophilization were similar, and that the amounts of the drugs finally released for 4 days were measured to be similar.

From the results, it could be seen that the shapes, physical properties and drug release profile of the microspheres undergoing the process of optimizing the recoverability after the lyophilization recovered into original states.

**Example 2: Preparation** of **doxorubicin liposome-containing microspheres** [reference example]

**2.1. Preparation of doxorubicin liposome**

Methanol and chloroform were mixed at a ratio of 1:1 to prepare a mixture solution, and soybean phosphatidylcholine that is a phospholipid was dissolved in the mixture solution. A thin lipid film remaining after a solvent was evaporated using a rotary vacuum evaporator was hydrated in a 250 mM ammonium sulfate solution. The size of the resulting liposome was regulated using an extruder, and the liposome was dialyzed four times in a 20 % (w/v) sucrose solution to form a transmembrane ammonium sulfate gradient from the inside to outside of the liposome. An aqueous doxorubicin solution was added to the previously prepared liposome suspension so that the concentration of doxorubicin was adjusted to 1 mg/ml, and doxorubicin was loaded into the liposome at $37 \pm 0.5$°C for 2 hours using a shaking incubator. Then, the unloaded doxorubicin was separated by centrifugation (at 2,000 g for 30 minutes).

**2.2. Measurement of drug loading efficiency and drug loading amount of doxorubicin liposome**

A sample was taken from the doxorubicin liposome, added into a container with a filter having a molecular weight cut-off (MWCO) of 100 K, and centrifuged at 2,000 g for 30 minutes to separate unloaded doxorubicin. The separated doxorubicin was measured for fluorescence at an excitation wavelength of 480 nm and an emission wavelength of 550 nm to calculate the amount of the doxorubicin. Thereafter, the drug loading efficiency and the drug loading amount of the doxorubicin-bound chitosan microspheres were calculated based on the measured values. As a result, it was revealed that the doxorubicin-bound chitosan microspheres had a drug loading efficiency of 56.74 % and a drug loading amount of 2.84 %, the chitosan microspheres bound to 10 mg of the doxorubicin-containing liposome had a drug loading efficiency of 60.73 % and a drug loading amount of 0.19 %, the chitosan microspheres bound to 30 mg of the doxorubicin-containing liposome had a drug loading efficiency of 91.09 % and a drug loading amount of 1.09 %, and the chitosan microspheres bound to 50 mg of the doxorubicin-containing liposome had a drug loading efficiency of 93.91 % and a drug loading amount of 1.88 %.

## Equation 1

$$\text{Drug loading efficiency} (\%) = \frac{\text{Total mass of doxorubicin} - \text{Unloaded doxorubicin}}{\text{Total mass of doxorubicin}} \times 100$$

## Equation 2

$$\text{Drug loading amount} (\%) = \frac{\text{Mass of loaded doxorubicin}}{\text{Mass of liposome} + \text{Mass of loaded doxorubicin}} \times 100$$

From the results, it could be seen that an exact amount of doxorubicin loaded into the liposome was able to be calculated, and thus that an exact amount of the drug included in the nano-carrier was able to be determined.

**2.3. Preparation of doxorubicin-containing chitosan microspheres and doxorubicin liposome-containing microspheres**

For comparison, doxorubicin-containing chitosan microspheres (Dox-CSMS) and doxorubicin liposome-containing microspheres were prepared using an emulsification-crosslinking method.

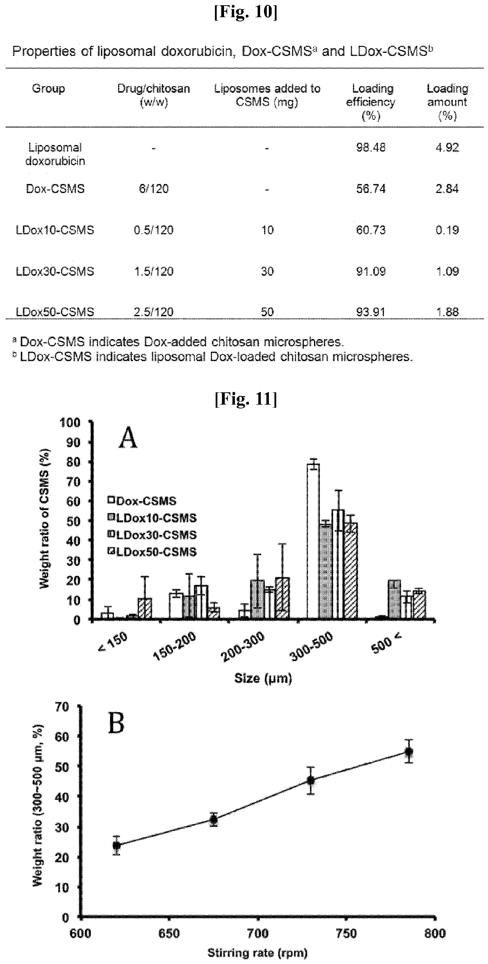

Chitosan was dissolved in a 5 % acetic acid solution in a glass vial until the concentration of chitosan reached 4 %. Therefore, doxorubicin and a doxorubicin liposome was added, and stirred for an hour. As shown in FIG. 10, 6 mg of doxorubicin and 120 mg of the chitosan microspheres were added to prepare the doxorubicin chitosan microspheres, 0.5 mg of the doxorubicin liposome and 120 mg of the chitosan microspheres were added to prepare the chitosan microspheres (LDox10-CSMS) containing 10 mg of the doxorubicin liposome, 1.5 mg of the doxorubicin liposome and 120 mg of the chitosan microspheres were added to prepare the chitosan microspheres (LDox30-CSMS) containing 30 mg of the doxorubicin liposome, and 2.5 mg of the doxorubicin liposome and 120 mg of the chitosan microspheres were added to prepare the chitosan microspheres (LDox50-CSMS) containing 50 mg of the doxorubicin liposome.

Next, 3 ml of each of the previously prepared doxorubicin chitosan solution and doxorubicin liposome chitosan solution was added to 30 ml of a solution obtained by mixing paraffin oil and petroleum ether at a ratio of 7:5, and 3 ml of sesquioleate was added as a surfactant to each container. Thereafter, the doxorubicin-containing chitosan microspheres, the liposome-containing chitosan microspheres onto which 10 mg of doxorubicin was loaded, the liposome-containing chitosan microspheres onto which 30 mg of doxorubicin was loaded, and the liposome-containing chitosan microspheres onto which 50 mg of doxorubicin was loaded were emulsified for 20 minutes while stirring at 620 rpm, 750 rpm, 785 rpm, and 850 rpm, respectively. In this case, the stirring rate was determined with reference to FIG. 11. FIG. 11A shows the results obtained by preparing the doxorubicin-containing chitosan microspheres and the doxorubicin liposome-containing chitosan microspheres and screening the chitosan microspheres whose size fell within a predetermined size range, using a sieve. In the present disclosure, the stirring rate was adjusted so that the microspheres having an optimum microsphere size of 300 to 500 $\mu$m used for embolization for treating liver cancer were obtained at as much amount as possible. FIG. 11B shows the results obtained by determining the optimized stirring rate to efficiently prepare the doxorubicin-containing chitosan microspheres whose size fell within 300 to 500$\mu$m. The size ratios of the respective indicated microspheres were obtained from the respective chitosan microspheres prepared at the optimized stirring rate. Representatively, the results corresponding to the chitosan microspheres containing 30 mg of the doxorubicin liposome are shown. Since the chitosan solution containing the doxorubicin liposome had a higher viscosity than the chitosan solution containing doxorubicin, the stirring rate had to increase further during an emulsification process to obtain a larger amount of the chitosan microspheres whose size fell within 300 to 500 $\mu$m. As shown in FIG. 11B, it could be seen that the chitosan microspheres whose size fell within 300 to 500 $\mu$m proportionally increased as the stirring rate increased.

From the results, it could be seen that the microspheres described herein were stably swollen in blood vessels since the microspheres were prepared to have an optimum size in the human body.

Subsequently, 4 ml of 25% glutaraldehyde-saturated toluene (GST) was added dropwise, and again stirred for an hour to crosslink the chitosan microspheres.

When the crosslinking was completed, the resulting chitosan microspheres were washed three times with petroleum ether, once with acetone, and three times with distilled water.

**2.4. Measurement of drug loading efficiency and drug loading amount of doxorubicin-containing chitosan microspheres and doxorubicin liposome-containing chitosan microspheres**

When the crosslinking was completed in the above-described process, an oily phase was taken to calculate the amount of the unloaded doxorubicin by measuring a degree of fluorescence. After the value was calculated, the drug loading efficiency of the chitosan microspheres was calculated using Equation 1, and the drug loading amount of the chitosan microspheres was calculated using the following Equation 3. The results are shown in FIG. 10. As a result, it was revealed that the Dox-CSMS had a drug loading efficiency of 56.74 % and a drug loading amount of 2.84%, the LDox10-CSMS had a drug loading efficiency of 60.73 % and a drug loading amount of 0.19 %, the LDox30-CSMS had a drug loading efficiency of 91.09 % and a drug loading amount of 1.09 %, and the LDox50-CSMS had a drug loading efficiency of 93.91 % and a drug loading amount of 1.88 %. From the results, it could be seen that the chitosan microspheres containing the doxorubicin liposome had a remarkably high drug loading efficiency.

Equation 3

$$\text{Drug loading amount (\%)} = \frac{\text{Mass of loaded doxorubicin}}{\text{Mass of chitosan} + \text{Mass of loaded doxorubicin}} \times 100$$

**2.5. *In vitro* release test**

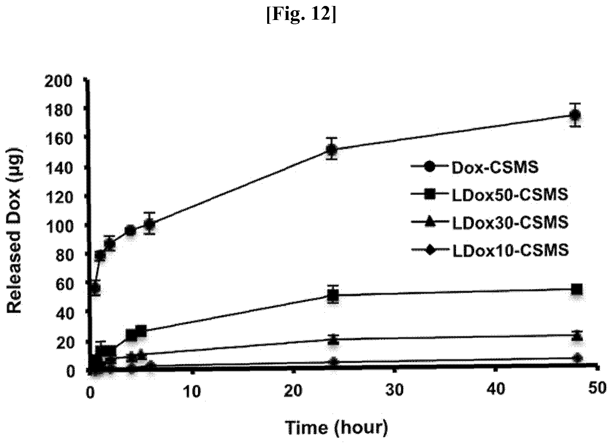

20 mg of each of the prepared doxorubicin-containing chitosan microspheres and doxorubicin liposome-containing chitosan microspheres were added into a dialysis bag, and the dialysis bag was put into a conical tube containing 30 ml of a PBS buffer (pH = 6.0, containing a lysozyme at 50,000 units). Each conical tube was put into a shaking incubator, and the release test conditions were set to be $37 \pm 0.5°C$ and 80 rpm. Thereafter, a release test was performed. In this case, 0.2 ml of a solution was taken at intervals of 30 minutes, 1 hour, 2 hours, 4 hours, 6 hours, 24 hours, and 48 hours, and degrees of fluorescence were measured to calculate the amount of the released doxorubicin. Immediately after 0.2 ml of the solution was taken, 0.2 ml of a solvent was added to conical tubes under the same conditions. The results are shown in FIG. 12. As a result, it was revealed that 140 $\mu$g of the drugs were released for 24 hours in the case of the doxorubicin chitosan microspheres (Dox-CSMS), 3 $\mu$g of the drugs were released for 24 hours in the case of the chitosan microspheres (LDox10-CSMS) containing 10 mg of the doxorubicin liposome, 18 $\mu$g of the drugs were released for 24 hours in the case of the chitosan microspheres (LDox30-CSMS) containing 30 mg of the doxorubicin liposome, and 44 $\mu$g of the drugs were released for 24 hours in the case of the chitosan microspheres (LDox50-CSMS) containing 50 mg of the doxorubicin liposome. Therefore, it was confirmed that the amount of the released drug was adjusted according to the amount of the added doxorubicin liposome. As a result it was confirmed that the release of the drug was controlled according to the amount of the added nano-carrier containing the drug.

It will be apparent to those skilled in the art that various modifications can be made to the above-described exemplary embodiments of the present disclosure without departing from the scope of the present disclosure. Thus, it is intended that the present disclosure covers all such modifications provided they come within the scope of the appended claims and their equivalents.

[Industrial Applicability]

When the method of lyophilizing microspheres for emboli according to one exemplary embodiment of the present disclosure is used, the microspheres having stable physical properties can be prepared even after the lyophilization. The microspheres prepared by the method have advantages in that they can be efficiently used for embolization since they are in a uniform spherical form, have a diameter of 500 $\mu$m or less, and rapidly recover an original shape before the lyophilization when inserted into the human body.

Also, since the chitosan microspheres to which the drug-containing nano-carrier is bound exhibits the specificity of precisely controlling the drug release, when the chitosan microspheres are used to exhibit a superior effect, compared to the conventional drug release microspheres. Therefore, the chitosan microspheres can be useful in controlling the selective release of the drug against a target tumor, and can be used as a new insert suitable for emboli, which is used for anticancer treatment and have an excellent embolic effect.

**Claims**

1. 1. A method of preparing microspheres for emboli, comprising:

    (a) adding trehalose to a microsphere suspension;
    (b) lyophilizing the suspension at a temperature in the range of -50 °C to -100 °C to prepare lyophilized microspheres; and
    (c) adding the lyophilized microspheres to water or a buffer and vortexing the microspheres.

2. The method of claim 1, wherein the microspheres are made of at least one material selected from the group consisting of chitosan, alginate, chitin, poly(N-isopropylacrylamide) (PNIPam), polyethylene glycol (PEG), poly(L-lactic acid) (PLLA), poly(D,L-lactic acid) (PDLLA), polyglycolic acid (PGA), polycaprolactone (PCL), polyhydroxyalkanoate, polydioxanone (PDS), polytrimethylene carbonate, poly(lactic acid-co-glycolic acid) (PLGA), poly(L-lactic acid-co-caprolactone) (PLCL), poly(glycolic acid-co-caprolactone) (PGCL), hyaluronic acid, chondroitin sulfate, dermatan sulfate, carboxymethyl cellulose, heparan sulfate, heparin, keratan sulfate, carboxymethyl hydroxyethyl cellulose, cellulose sulfate, cellulose phosphate, carboxymethyl guar, carboxymethyl hydroxypropyl guar, carboxymethyl hydroxyethyl guar, xanthan gum, Gellan gum, Welan gum, Rhamsan gum, agarose, furcellaran, pectin, gum arabic, gum tragacanth, carrageenan, starch phosphate, starch succinate, glycoaminoglycan, a polysaccharide, a polypeptide, an acrylamide, N-vinylpyrrolidone, dimethylacrylamide, acrylic acid, methacrylic acid, anhydric maleic acid, vinyl sulfonate, styrenecarboxylic acid, 2-acrylamido-2-methyl-propanesulfonic acid, vinylphosphonic acid, 2-methylacryloyloxyethyl sulfonic acid, gelatin, and collagen.

3. The method of claim 1, wherein operation (c) comprises further adding a contrast agent, preferably a magnetic resonance imaging (MRI) contrast agent, a computed tomography (CT) contrast agent, a single photon emission computed tomography (SPECT) contrast agent, a positron emission tomography (PET) contrast agent, a bioluminescence (BL) contrast agent, an optical contrast agent, an X-ray contrast agent, or an ultrasonic contrast agent.

4. The method of claim 1, wherein the vortexing is performed for 5 minutes to 20 minutes.

5. The method of claim 1, wherein a drug is able to be released from the microspheres prepared by the method.

6. The method of claim 5, wherein the drug is a drug for treating cancer, preferably wherein the drug is doxorubicin.


**Patentansprüche**

1. Verfahren zur Herstellung von Mikrokügelchen für Emboli, umfassend:

    a) Zugabe von Trehalose zu einer Mikrokügelchen-Suspension;
    b) Lyophilisieren der Suspension bei einer Temperatur im Bereich von -50 °C bis -100 °C zur Herstellung lyophilisierter Mikrokügelchen und
    c) Zugabe der lyophilisierten Mikrokügelchen zu Wasser oder einem Puffer und Verwirbelung der Mikrokügelchen.

2. Verfahren nach Anspruch 1, wobei die Mikrokügelchen aus mindestens einem Material hergestellt sind, das aus der Gruppe ausgewählt ist, die aus Chitosan, Alginat, Chitin, Poly(N-isopropylacrylamid) (PNIPam), Polyethylenglykol (PEG), Poly(L-milchsäure) (PLLA), Poly(D,L-milchsäure) (PDLLA), Polyglykolsäure (PGA), Polycaprolacton (PCL), Polyhydroxyalkanoat, Polydioxanon (PDS), Polytrimethylencarbonat, Poly(milchsäure-co-glykolsäure) (PLGA), Poly(L-milchsäure-co-caprolacton) (PLCL), Poly(glykolsäure-co-caprolacton) (PGCL), Hyaluronsäure, Chondroitinsulfat, Dermatansulfat, Carboxymethylcellulose, Heparansulfat, Heparin, Keratansulfat, Carboxymethylhydroxyethylcellulose, Cellulosesulfat, Cellulosephosphat, Carboxymethylguar, Carboxymethylhydroxypropylguar, Carboxymethylhydroxyethylguar, Xanthangummi, Gellangummi, Welangummi, Rhamsangummi, Agarose, Furcellaran, Pektin, Gummi arabicum, Tragacanthgummi, Carrageenan, Stärkephosphat, Stärkesuccinat, Glykoaminoglykan, einem Polysaccharid, einem Polypeptid, einem Acrylamid, N-Vinylpyrrolidon, Dimethylacrylamid, Acrylsäure, Methacrylsäure, Maleinsäureanhydrid, Vinylsulfonat, Styrolcarbonsäure, 2-Acrylamido-2-methylpropansulfonsäure, Vinylphosphonsäure, 2-Methylacryloyloxyethylsulfonsäure, Gelatine und Kollagen besteht.

3. Verfahren nach Anspruch 1, wobei der Vorgang (c) weiter die Zugabe eines Kontrastmittels, vorzugsweise eines Kontrastmittels für die Magnetresonanztomographie (MRI), eines Kontrastmittels für die Computertomographie (CT), eines Kontrastmittels für die Einzelphotonen-Emissions-Computertomographie (SPECT), eines Kontrastmittels für die Positronen-Emissions-Tomographie (PET), eines Kontrastmittels für die Biolumineszenz (BL), eines optischen Kontrastmittels, eines Röntgenkontrastmittels oder eines Ultraschallkontrastmittels umfasst.

**4.** Verfahren nach Anspruch 1, wobei die Wirbelbildung für 5 Minuten bis 20 Minuten durchgeführt wird.

**5.** Verfahren nach Anspruch 1, wobei ein Arzneimittel befähigt ist, aus den durch das Verfahren hergestellten Mikrokügelchen freigesetzt zu werden.

**6.** Verfahren nach Anspruch 5, wobei das Arzneimittel ein Arzneimittel zur Behandlung von Krebs ist, vorzugsweise das Arzneimittel Doxorubicin.

**Revendications**

**1.** Procédé de préparation de microsphères pour emboles, comprenant :

(a) ajouter du tréhalose à une suspension de microsphères ;
(b) lyophiliser la suspension à une température comprise entre -50°C et -100°C pour préparer des microsphères lyophilisées ; et
(c) ajouter les microsphères lyophilisées à de l'eau ou à un tampon et soumettre les microsphères à un vortex.

**2.** Procédé selon la revendication 1, dans lequel les microsphères sont constituées d'au moins un matériau choisi dans le groupe constitué par le chitosan, l'alginate, la chitine, le poly(N-isopropylacrylamide) (PNIPam), le polyéthylène glycol (PEG), le poly(acide lactique L) (PLLA), le poly(acide lactique D, L) (PDLLA), l'acide polyglycolique (PGA), la polycaprolactone (PCL), le polyhydroxyalcanoate, la polydioxanone (PDS), le carbonate de polytriméthylène, le poly(acide lactique-co-glycolique) (PLGA), le poly(acide lactique L-co-caprolactone) (PLCL), le poly(acide glycolique-co-caprolactone) (PGCL), l'acide hyaluronique, le sulfate de chondroïtine, le sulfate de dermatane, la carboxyméthylcellulose, le sulfate d'héparane, l'héparine, le sulfate de kératane, la carboxyméthyl-hydroxyéthyl-cellulose, le sulfate de cellulose, le phosphate de cellulose, le carboxyméthyl-guar, le carboxyméthyl-hydroxypropyl-guar, le carboxyméthyl-hydroxyéthyl-guar, la gomme de xanthane, la gomme de gellane, la gomme de welane, la gomme rhamsane, l'agarose, la furcellarane, la pectine, la gomme arabique, la gomme adragante, le carraghénane, le phosphate d'amidon, le succinate d'amidon, le glycoaminoglycane, un polysaccharide, un polypeptide, un acrylamide, la Nvinylpyrrolidone, le diméthylacrylamide, l'acide acrylique, l'acide méthacrylique, l'acide maléique anhydre, le vinylsulfonate, l'acide styrène-carboxylique, l'acide 2-acrylamido-2-méthylpropanesulfonique, l'acide vinylphosphonique, l'acide 2-méthylacryloyloxyéthylsulfonique, la gélatine et le collagène.

**3.** Procédé selon la revendication 1, dans lequel l'opération (c) comprend en outre l'ajout d'un agent de contraste, de préférence un agent de contraste d'imagerie par résonance magnétique (IRM), un agent de contraste de tomodensitométrie (CT), un agent de contraste de tomographie d'émission monophotonique (SPECT), un agent de contraste de tomographie par émission de positons (PET), un agent de contraste de bioluminescence (BL), un agent de contraste optique, un agent de contraste aux rayons X ou un agent de contraste ultrasonore.

**4.** Procédé selon la revendication 1, dans lequel le vortex est effectué pendant 5 minutes à 20 minutes.

**5.** Procédé selon la revendication 1, dans lequel un médicament peut être libéré des microsphères préparées par le procédé.

**6.** Procédé selon la revendication 5, dans lequel le médicament est un médicament destiné au traitement du cancer, de préférence dans lequel le médicament est la doxorubicine.

[Fig. 1]

[Fig. 2]

[Fig. 3]

[Fig. 4]

[Fig. 5]

[Fig. 6]

[Fig. 7]

**[Fig. 8]**

**[Fig. 9]**

## [Fig. 10]

Properties of liposomal doxorubicin, Dox-CSMS[a] and LDox-CSMS[b]

| Group | Drug/chitosan (w/w) | Liposomes added to CSMS (mg) | Loading efficiency (%) | Loading amount (%) |
|---|---|---|---|---|
| Liposomal doxorubicin | - | - | 98.48 | 4.92 |
| Dox-CSMS | 6/120 | - | 56.74 | 2.84 |
| LDox10-CSMS | 0.5/120 | 10 | 60.73 | 0.19 |
| LDox30-CSMS | 1.5/120 | 30 | 91.09 | 1.09 |
| LDox50-CSMS | 2.5/120 | 50 | 93.91 | 1.88 |

[a] Dox-CSMS indicates Dox-added chitosan microspheres.
[b] LDox-CSMS indicates liposomal Dox-loaded chitosan microspheres.

## [Fig. 11]

[Fig. 12]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100478227
- KR 101157260
- US 20090274762 A
- US 2011293672 A
- KR 101058196

**Non-patent literature cited in the description**

- **BEAUJEUX et al.** Trisacryl gelatin microspheres for therapeutic embolization, II: Preliminary Clinical evaluation in tumors and arteriovenous malformations. *Am J Neuroradiol,* 1996, vol. 17, 541-548